# EUROPEAN PATENT SPECIFICATION

(11) **EP 1 525 469 B1**
(45) Date of publication and mention of the grant of the patent: **16.08.2006**
(21) Application number: 03771499.5
(22) Date of filing: 30.07.2003
(51) Int. Cl.: G01N 33/38, G01N 21/35

(54) **ANALYTICAL SYSTEM AND METHOD FOR MEASURING AND CONTROLLING A PRODUCTION PROCESS**
ANALYTISCHES SYSTEM UND VERFAHREN ZUM MESSEN UND STEUERN EINES HERSTELLUNGSVERFAHRENs
SYSTEME ET PROCEDE D'ANALYSE POUR MESURER ET CONTROLER UN PROCEDE DE PRODUCTION

(30) Priority: 30.07.2002 NL 1021182
(43) Date of publication of application: 27.04.2005
(73) Proprietor: Centrum voor Technische Informatica B.V., 9711 EK Groningen (NL)
(72) Inventor: DALSTRA, Joop, NL-9753 KH Haren (NL)
(74) Representative: van Westenbrugge, Andries
(86) International application number: PCT/NL2003/000547
(87) International publication number: WO 2004/011935

(56) References cited:
- EP-A1- 0 643 297
- WO-A1-02/28954
- US-A- 5 220 403

## Description

The present invention relates to an analytical system for analysing and monitoring a production process for glass products according to the preamble of claim 1.

A system of this type is disclosed in the patent application EP 1020703 A2. That patent application describes a method and apparatus of measuring wall thickness of hollow glass articles, such as molded glass containers having interior and exterior wall surfaces, includes the steps of measuring intensity of radiation emitted by the article at a first wavelength at which intensity varies as a function of both temperature at the surfaces and wall thickness between the surfaces, and at a second wavelength at which intensity varies as a function of temperature at the surface substantially independent of wall thickness between the surfaces. Since the first intensity measurement is a function of both wall thickness and temperature, while the second intensity measurement is a function solely of surface temperature, wall thickness between the surfaces can determined as a combined function of the first and second intensity measurements. When a glass article shows a deviated wall thickness, the glass article is rejected from the process.

Publication "A guide to radiation thermal meters", Glass Technology Intemational-2/1998, describes a thermal imager arranged to detect infrared radiation from hot glass during a glass forming process. A detector is used at a selected band around 5 microns, which can measure the surface temperature of the glass. Furthermore, a thermometer operational at a wave length of 1 micron is used to measure the bulk of the glass.

Publication "Non-contact infrared sensor ensure accurate temperature monitoring", Glass International-January/February 2001, describes infrared sensors arranged to measure both the surface and beneath the surface of a glass object by using a wave length, such as 1.0 micrometer or 2.2 micrometer.

Publication US 4,759,072 describes a method and apparatus for detection of surface defects of hot metal bodies e.g. hot-rolled steel sheet. A video signal is derived from radiation spontaneously radiated from a target area of the hot metal surface. A near-infrared region of the radiation is used in producing the video signal.

One aim of the present invention is to provide an alternative system for monitoring a production process for glass products..

In order to achieve this aim, the present invention relates to an analytical system of the type mentioned in the preamble, characterised in that the processor is arranged to carry out the following steps:
(a) subdividing an image of the glass products into at least two measurement regions;
(b) determining average intensity values for the different measurement regions for consecutive glass products;
(c) determining, for at least two measurement regions, a current average value from the average intensity values determined for a number of consecutively shaped glass products over time;
(d) recording, for each of the at least two measurement regions, any deviation between the current intensity or the current average intensity and a reference value;
(e) comparing any deviations between the at least two measurement regions;
(f) generating an error signal in the event of any deviations.

By analysing the deviations between the at least two measurement regions it is possible to determine whether a change in glass wall thickness has occurred or whether a change in temperature has occurred. In this context change signifies a change with respect to the previous glass products and glass products produced in the past.

Infrared light with long wavelengths is completely absorbed in the interior of the glass wall. This is not the case with NIR radiation. NIR radiation essentially originates from the interior of the glass wall and the measured amount of NIR radiation is thus correlated to the amount of heat in the interior of the glass wall. Preferably, the infrared-sensitive measurement system is sensitive to wavelengths of between 900 and 2800 nanometres. It has been found that optimum results are obtained at these wavelengths.

In one embodiment of the invention the measurement system comprises at least one infrared sensor and at least one Near Infra Red filter. Preferably, the transmission characteristics of the Near Infra Red filter are dependent on the colour and the specific material composition of the glass products. This ensures optimum measurement sensitivity.

In another aspect of the invention, there is provided an analytical system according to claim 7.

By determining an optimum fit curve and taking this as reference curve, current machine plots can be compared with this. This can be performed individually for each measurement region. Deviations in the machine plots compared with the fit curve provide information on errors in the shaping process. By means of this analytical method both the quality of the shaping process and also the quality of the glass products can be monitored. If the measured intensities are precisely on the cooling plot, the glass products will have the same quality.

Furthermore, the present invention relates to a method for analysing and monitoring a shaping process for glass products, as described in Claim 9. By measuring radiation in the Near Infra Red region the heat distribution in the interior of the glass wall can be determined, which offers possibilities for novel analytical methods.

Although the intensity of the measured radiation is dependent on the temperature distribution, the amount of glass and the material properties, changes in the thickness of the glass wall can readily be determined by means of this method. By comparing the deviations from average intensities in two measurement regions it is possible to determine whether the change in the measured radiation is to be ascribed to a change in the thickness of the glass wall or to a change in the temperature of the glass. This analytical method will be explained in more detail in the description of the figures.

In another aspect, the invention relates to a method according to Claim 14. With the aid of this method the correct settings for the shaping process can rapidly be determined and a setup time in the case of a change in production is shortened. Moreover, deviations in the current machine plots for the different measurement regions can be used to analyse faults in individual sub-processes of the shaping process.

Further advantages and characteristics of the present invention will become clear on the basis of a description of a number of embodiments, reference being made to the appended drawings, in which:
Fig. 1 shows a production process from the state of the art,
Fig. 2 shows, diagrammatically, a glass shaping machine and a measurement system from the state of the art,
Fig. 3 shows, diagrammatically, a measurement system according to the invention,
Fig. 4 shows an example of a subdivision of the glass products into measurement regions.
Fig. 5 is a graph showing the change in the average intensity of two measurement regions and a reference value,
Fig. 6 is a graph showing the change in the average intensity of two measurement regions and a reference value,
Fig. 7 is a graph of a so-called machine plot,
Fig. 8 is a graph of a so-called machine plot with a fit curve.

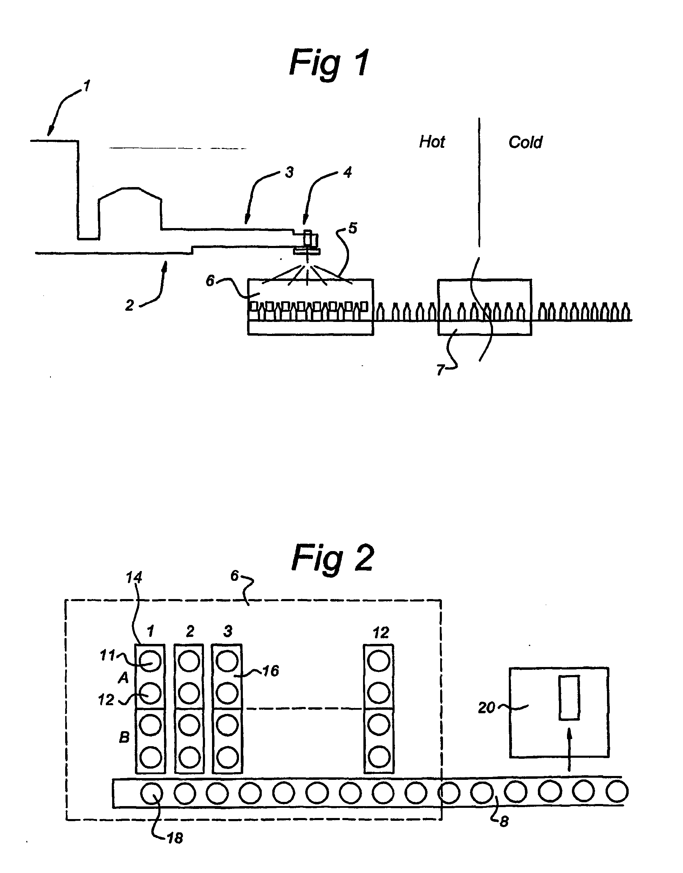

Figure 1 shows a known production process for hollow glass products in which various process steps can be recognised. In a smelter 1 recycled glass fragments, mixed with basic raw material and additives, are remelted to give liquid glass. The molten glass flows from the smelter 1 via one or more channels 2 ("forehearth") to a feeder 3. Downstream of the feeder 3 the stream of glass is cut into glass gobs in a gob forming process 4. The glass gobs are then fed via a gob transport 5 to an Independent Section (IS) machine 6.

The IS machine 6 in which the shaping process takes place is shown in more detail in Figure 2. In the IS machine 6 each glass gob is shaped into a product. The shaping process is, for example, carried out with the aid of two moulds. The gob first falls into a first mould (the so-called blank mould 11) where, depending on the shaping process, the first stage of the product is blown or pressed. This first stage of the product, also termed the parison, is then transported to a second mould (the so-called blow mould 12), where the parison is blown to give the final shape of a glass product 18. The section 16 with the two moulds is also termed a station. The IS machine 6 consists of several parallel sections 14. Each section 14 can, in turn, consist of several stations 16 which are able to produce products independently of one another. The blown glass products 18 are placed one after the other on a conveyor belt 8 and fed to a cooling oven 7; see Figure 1. In the cooling oven 7 the products are heated to above the so-called annealing point of the glass. The products are rendered stress-free by this means. The products can now cool and can be packed and transported to their destination. The section of the production process downstream of the cooling oven is also termed the "cold" section of the production area. During production a wide variety of faults which have an adverse effect on the quality of the glass product can arise in every process step. Consequently it is necessary for the process variables of each process step to be set within very narrow tolerances and to be monitored. These process settings are dependent on the type of end product and have to be re-adjusted for the production of a different type of product (the so-called product change). An end product 18 of good quality has the correct dimensions, has a uniform glass thickness, does not have any cracks, has an even colour and has a high degree of glass purity. Glass purity means that the glass must be free from all sorts of material foreign to glass, such as grit, air bubbles, metals and contamination.

In order to be able to offer the customer glass products 18 of a consistently high quality, the glass products are inspected to determine their quality. To prevent information from the shaping process being lost by the annealing process, currently use is made of an infrared measurement system 20, see Figure 2, which measures the thermal radiation by the glass product 18 before the glass product enters the cooling oven 7. The information obtained by the infrared measurement system 20 can be used to monitor the quality of the glass products 18 and the process. The known measurement system 20 has the disadvantages mentioned above.

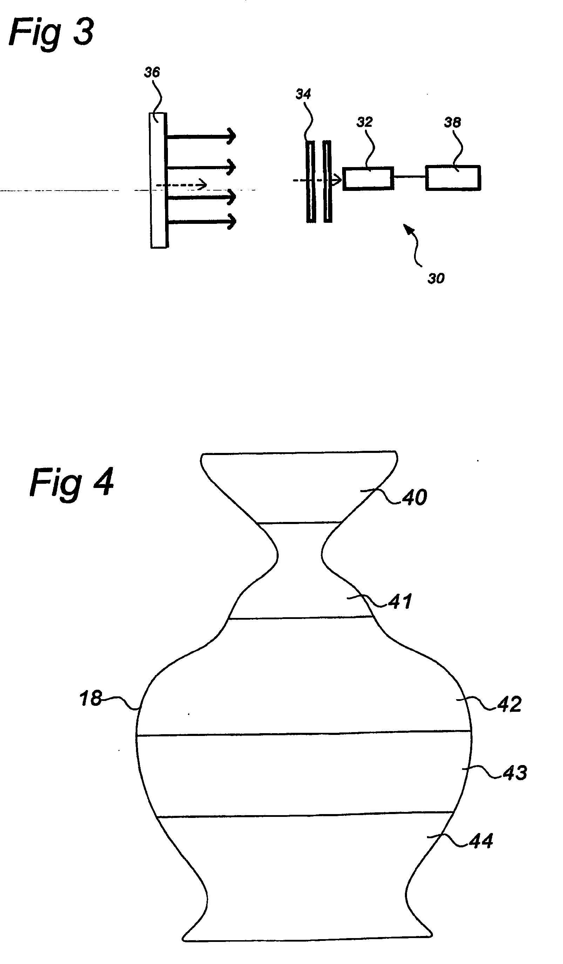

Figure 3 shows, diagrammatically, a novel measurement system 30 according to one embodiment of the invention. The measurement system 30 comprises a filter system 34, at least one infrared sensor 32 and a digital processor 38. The filter system 34 allows selective transmission only of infrared radiation in the Near Infra Red (NIR) region, that is to say radiation having a wavelength of between 600 and 5000 nanometres. Thermal radiation in the NIR region mainly originates from the interior of a glass wall 36. Preferably the filter system is so equipped that it allows the transmission of radiation in the wavelength range of 900 to 2800 nanometers, depending on the specific composition of the glass. In Figure 3 the NIR radiation is shown by the thin dashed arrows. The digital processor 38 is arranged to analyse a heat distribution in a glass product on the basis of measurement data. This can take place in various ways, which are described in the embodiments below.

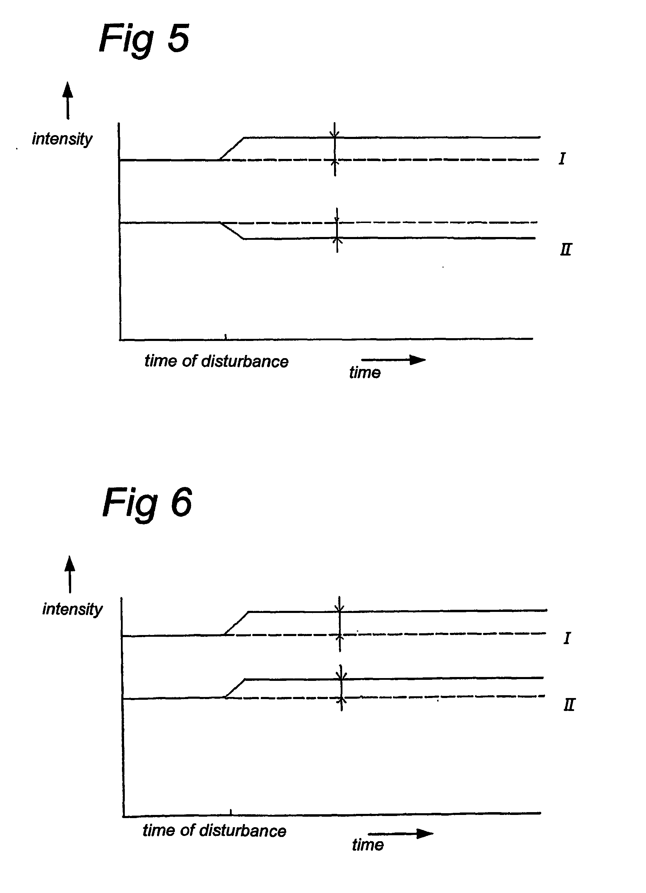

In one embodiment the digital processor 38 is arranged to subdivide the heat distribution obtained for a glass product into so-called measurement regions 40, 41, 42, 43, 44; see Figure 4. These can be a number of strips that subdivide the image of the glass product 18 into horizontal measurement regions 40, 41, 42, 43, 44 (see Figure 4), but a different form of measurement regions 40, 41, 42, 43, 44 is also possible. The number of measurement regions 40, 41, 42, 43, 44 is two or more. The number of measurement regions is not relevant, but more detailed information on the shaping process is obtained with a larger number of measurement regions. The measured intensities of the radiation are preferably averaged over each measurement region 40, 41, 42, 43, 44. The current average value thus obtained is compared with a reference value. This reference value is determined by the cooling curve originating from the measurement region or by another statistical calculation such as, for example, the running average. If the current average value is greater than the reference value the difference is 'positive'; see Figure 5. If the average value is lower than its reference value this difference is then 'negative'.

This analysis is carried out for each measurement region 40, 41, 42, 43, 44 set. If there are measurement regions 40, 41, 42, 43, 44 that display a difference and have an opposite sign the change is to be ascribed to a change in the glass thickness; see Figure 5. Explanation: each glass product is formed from a glass gob. The gobs have a constant weight and volume. The quantity of glass per product is thus constant. If, as a result of a disturbance in the process, a thinner glass wall is produced somewhere in the product, for example in the base section, the glass wall thickness in another measurement region 40, 41, 42, 43, 44 of the product then has to increase. The measurement regions 40, 41, 42, 43, 44 with a thinner glass wall will emit less radiation; the measurement regions 40, 41, 42, 43, 44 with a thicker glass wall will emit more radiation. The change cannot be ascribed to a change in the material properties since the glass for the products comes from the same furnace.

Figure 6 shows a graph with a different change in the average intensity of the measurement regions 40, 41, 42, 43, 44. As a result of a disturbance in the process a deviation in the radiation occurs. Because in this case the measured deviation has a matching sign there has been a change in the glass wall temperature. Explanation: each glass product is formed from a glass gob. The gobs have a constant weight and volume. The quantity of glass per product is thus constant. If, as the result of a disturbance in the process, the temperature of the glass product 18 rises, those parts of the glass product 18 that are hotter will then all emit more radiation. Since the thickness of the glass wall has not changed, the deviations in the relevant measurement regions 40, 41, 42, 43, 44 will all have a matching sign for the difference. The change cannot be ascribed to a change in the material properties since the glass for the glass products 18 comes from the same smelter 1.

Each section 14 of the IS machine 6 consists of one or more stations 16. Each station 16 can produce a glass product 18 independently of the other sections 14. The glass products 18 that have just been formed are in a fixed sequence on the conveyor belt 8. Depending on the section 14 from which they have been produced, the glass products 18 all have a different cooling time. This is the time between the end of the shaping process and the time when the product passes by the measurement system 30.

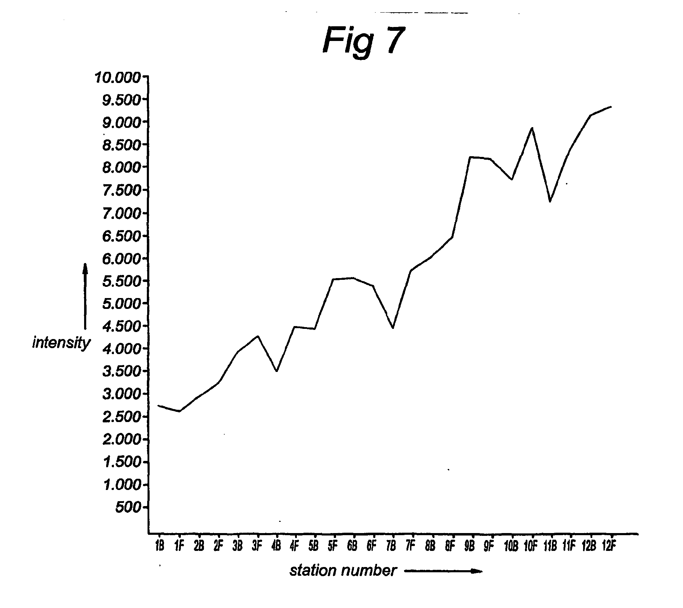

Because the invention is preferably synchronised over time with the IS machine 6, the station 16 from which the glass product 18 originates is known for each glass product 18. In Figure 7 the measured intensity is plotted against the various stations 16 for one specific measurement region 40, 41, 42, 43, 44. The names of the stations (B and F) associated with the various sections ('1', .. '12') are plotted along the X axis. Stations 16 that are closer to the measurement system 30 have a shorter cooling time and thus also have a higher radiation level at the point in time when they pass by the measurement system 30. Thus, it can be seen in Figure 7 that a glass product 18 from station '12B', which is close to the measurement system 30 (see also Figure 2), is hotter than a glass product 18 from station '1B', which is far away from the measurement system 30. The graph obtained is termed the IS machine plot.

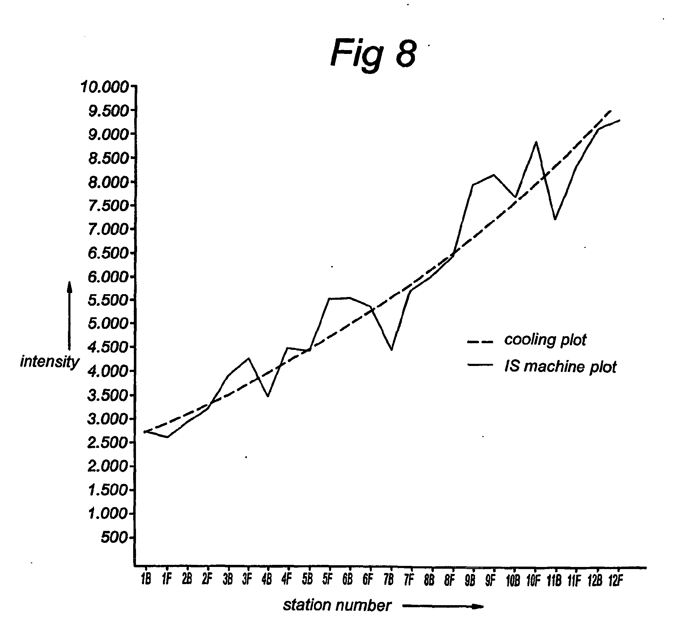

In Figure 8 an exponential curve that has been calculated with the aid of the "least squares" or a similar method has been drawn through the measurement points from Figure 7. This curve is termed the cooling curve. If all glass products formed have the same glass wall thickness, temperature distribution and material characteristics after their final shaping process, the measurement points of the IS machine plot will then lie precisely on the cooling curve. The glass products 18 will all be of the same quality. If, however, a disturbance occurs in a process step for a specific section 14 (and thus station), the products originating from this section 14 will be affected by a change in quality. The temperature distribution and/or the glass wall thickness will change. As a result the IS machine plot will display a deviation with respect to the cooling curve. If the measured intensities are on the cooling curve, the glass products 18 will then be of the same quality. The conclusion is then also that the cooling curve can be used as a reference value for the shaping process. The values of IS machine setting parameters associated with a specific cooling curve for a glass product 18 can serve as reference values for the future production of the glass product 18.

When another type of glass product has to be produced, all setting parameters for the shaping process will then have to be adjusted. To appreciably shorten this adjustment time and to reduce the large amount of guesswork, the (already known) setting parameters from the cooling curve for the glass product are immediately used as reference value. The settings for the shaping process are now so adjusted that the IS machine plot becomes identical to the cooling curve. In this way all glass products 18 acquire the same quality as in the previous production.

By recording any deviations between a current IS machine plot and the cooling curve it is possible to indicate an error in the shaping process and to determine in which process step this error has occurred. Preferably, the IS machine plots and the cooling curves are determined for all set measurement regions 40, 41, 42, 43, 44 for the current process. The calculated cooling curves are used as reference values for each station. If a deviation occurs in the IS machine plot with respect to the cooling curve then the following situations can have arisen:
Situation A: The deviation applies to all sections and the new calculated cooling curves have been shifted up or down compared with the existing cooling curves, but the shape of the cooling curve has remained virtually the same.
Analysis A: A deviation has occurred for all sections. This means that a fault has occurred in the entire IS machine, such as, for example, the cooling capacity for all sections, or a fault has occurred in the process steps upstream of the IS machine in the feeder, forehearth and smelter. Furthermore, the fault is solely of a thermal nature.
Explanation: A station in a section can produce glass products 18 independently of other sections. If a deviating radiation pattern with respect to the cooling curve (the reference) is determined, the fault must then have been caused by a common factor. This is either a common factor in the IS machine 6 (such as the temperature, humidity of the cooling air in the IS machine 6) or a common factor in the process steps upstream of the IS machine 6. That is to say the temperature, material characteristics in the feeder, forehearth and smelter 1. The shape of the cooling curves has remained virtually the same. This means that the cooling rate of the products has also remained the same. It can thus be concluded that the initial temperature after the final production step in the IS machine 6 has increased or decreased for all sections 14 and that both the glass distribution and the material characteristics have remained the same.
Situation B: The deviation applies to all sections and the new calculated cooling curves have shifted up or down compared with the existing cooling curves but the shape of the cooling curve has also changed.
Analysis B: Once again there is a fault in all sections. Thus, the fault that has occurred must be a common factor. Because the shape of the cooling curves has changed, it can be concluded that the material characteristics of the glass have changed and that consequently the glass distribution has also changed.
Explanation: The shape of the cooling curves is dependent on the glass thickness of the glass wall and on the material characteristics but not on the initial temperature in the glass wall of the product. Since the quantity of glass remains virtually constant (gob), the deviation that has occurred simultaneously for all sections 14 must have been caused by a change in the material characteristics.
Situation C: A deviation occurs only for the stations 16 that have a common gob forming process.
Analysis C: If a deviation occurs in the IS machine plot compared with the cooling curve only for the stations 16 that have a common gob forming process, the disturbance is then caused in the gob forming process. If the average intensity of the stations with a deviation is higher or lower, the weight of the gob is then higher or lower.
Situation D: The deviation in the IS machine plot with respect to the cooling curve relates only to a single station 16.
Analysis D: A fault has occurred only in the station 16 concerned. Only those process components in the station can be the cause of the fault.

The embodiments described above are intended solely to serve as example and in no way are intended to restrict the invention. A person skilled in the art will rapidly be able to devise other embodiments, such as, for example, the measurement of only a single bottle as a function of time so that a cooling curve can be obtained by this means. The IS machine 6 can also be made up of a different composition of sections 14 and stations 16, as a result of which the analytical methods proceed somewhat differently. It will also be clear to a person skilled in the art that the digital processor 38 can be replaced by any other suitable processor. The processor 38 can be constructed using analogue, digital or software techniques or any desired combination thereof. The processor 38 can also consist of various sub-processes, optionally in a master-slave relationship. The processor does not necessarily have to be close to the rest of the system but can, for example, communicate with the measurement system via remote communication.

## Claims

1. Analytical system for analysing and monitoring a production process for glass products, the production process comprising a shaping process and a cooling process and the analytical system comprising an infrared-sensitive measurement system and a processor communicating therewith, the infrared-sensitive measurement system being equipped to measure infrared radiation originating from hot glass products immediately after the shaping process for the glass products and the processor being arranged to determine a heat distribution in the glass products on the basis of information determined by the measurement system, said infrared-sensitive measurement system (30) being sensitive only to radiation in the Near Infra Red (NIR) region, originating from an interior of a wall of the glass products, **characterised in that** the processor (38) is arranged to carry out the following steps:
(a) subdividing an image of the glass products (18) into at least two measurement regions (40, 41, 42, 43, 44);
(b) determining average intensity values for the different measurement regions for consecutive glass products (18);
(c) determining, for at least two measurement regions, a current average value from the average intensity values determined for a number of consecutively shaped glass products (18) over time;
(d) recording, for each of the at least two measurement regions, any deviation between the current intensity or the current average intensity and a reference value;
(e) comparing any deviations between the at least two measurement regions;
(f) generating an error signal in the event of any deviations.

2. Analytical system according to Claim 1, **characterised in that** the infrared-sensitive measurement system (30) is sensitive to wavelengths of between 900 and 2800 nanometers.

3. Analytical system according to one of the preceding claims, **characterised in that** the infrared-sensitive measurement system (30) comprises at least one infrared sensor (32) and at least one Near Infra Red filter (34).

4. Analytical system according to Claim 3, **characterised in that** the transmission characteristics of the Near Infra Red filter (34) are dependent on the colour and the specific material composition of the glass products.

5. Analytical system according to Claim 1, **characterised in that** the error signal is indicative of a deviating glass thickness if a positive deviation occurs in a first measurement region and a negative deviation occurs in a second measurement region.

6. Analytical system according to Claim 1, **characterised in that** the error signal is indicative of a deviating glass temperature if a positive deviation occurs for all measurement regions or a negative deviation occurs for all measurement regions.

7. Analytical system for analysing and monitoring a production process for glass products, the production process comprising a shaping process and a cooling process and the analytical system comprising an infrared-sensitive measurement system and a processor communicating therewith, the infrared-sensitive measurement system being equipped to measure infrared radiation originating from hot glass products immediately after the shaping process for the glass products and the processor being arranged to determine a heat distribution in the glass products on the basis of information determined by the measurement system, said infrared-sensitive measurement system (30) being sensitive only to radiation in the Near Infra Red (NIR) region, originating from an interior of a wall of the glass products, **characterised in that** the processor (38) is arranged to carry out the following steps:
(a) subdividing an image of the glass products (18) into at least two measurement regions (40, 41, 42, 43, 44);
(b) determining average intensity values for the different measurement regions for consecutive glass products (18),
wherein the processor (38) is arranged to carry out the following steps for at least one measurement region:
(c) determining a machine plot by plotting a graph of the average intensity values as a function of the consecutive glass products (18), i.e. stations (14);
(d) determining a cooling plot by means of an optimum fit curve;
(e) recording any deviations between a current machine plot and the cooling plot;
(f) generating an error signal in the event of any deviations.

8. Analytical system according to Claim 7, **characterised in that** the error signal contains information on a possible cause during the shaping process.

9. Method for analysing and monitoring a production process for glass products, comprising:
a) providing measurement means for determining a heat distribution in hot glass produce said measurement means (30) being sensitive only to radiation from the Near Infra Red region, originating from an interior of a wall of the glass products.
b) measuring infrared radiation originating from the hot glass products before these enter a cooling oven;
c) determining a heat distribution in glass products on the basis of the infrared radiation measured;
(d) subdividing an image of the glass products (18) into at least two measurement regions (40, 41, 42, 43, 44);
(e) determining average intensity values for the different measurement regions for consecutive glass products (18);
(f) determining, for at least two measurement regions, a current average value from the average intensity values determined for a number of consecutively shaped glass products (18);
(g) recording, for each of the at least two measurement regions, any deviation between the current average intensity and a reference value;
(h) comparing any deviations between the at least two measurement regions;
(i) generating an error signal in the event of any deviations.

10. Method according to Claim 9, **characterised in that** the measurement means (30) are sensitive only to wavelengths between 900 and 2800 nanometres.

11. Method according to Claim 9, **characterised in that** the measurement means (30) comprise at least one infrared sensor (32) and at least one Near Infra Red filter (34).

12. Method according to Claim 9, **characterised in that** the error signal is indicative of a deviating glass thickness if a positive deviation occurs in a first measurement region and a negative deviation occurs in a second measurement region.

13. Method according to Claim 9, **characterised in that** the error signal is indicative of a deviating glass temperature if a positive deviation occurs for all measurement regions or a negative deviation occurs for all measurement regions.

14. Method for analysing and monitoring a production process for glass products, comprising:
a) providing measurement means for determining a heat distribution in hot glass products said measurement means (30) being sensitive only to radiation from the Near Infra Red region, originating from an interior of a wall of the glass products.
b) measuring infrared radiation originating from the hot glass products before these enter a cooling oven;
c) determining a heat distribution in glass products on the basis of the infrared radiation measured;
(d) subdividing an image of the glass products (18) into at least two measurement regions (40, 41, 42, 43, 44);
(e) determining average intensity values for the different measurement regions for consecutive glass products (18);
(f) determining a machine plot by plotting a graph of the average intensity values as a function of the consecutive glass products (18), i.e. stations (14);
(g) determining a cooling plot by means of an optimum fit curve;
(h) recording any deviations between a current machine plot and the cooling plot;
(i) generating an error signal in the event of any deviations.

## Patentansprüche

1. Analytisches System zum Analysieren und Überwachen eines Produktionsprozesses für Glaserzeugnisse, wobei der Produktionsprozess einen Formprozess und einen Kühlprozess umfasst, und das analytische System ein infrarotempfindliches Messsystem und einen Prozessor umfasst, der zwischen diesen kommuniziert, wobei das infrarotempfindliche Messsystem ausgerüstet ist, um Infrarotstrahlung, die aus heißen Glaserzeugnissen unmittelbar nach dem Formprozess für die Glaserzeugnisse ausgeht, zu messen; und der Prozessor so angeordnet ist, um eine Wärmeverteilung in den Glaserzeugnissen auf der Basis von Informationen zu bestimmen, die durch das Messsystem festgelegt werden, das infrarotempfindliche Messsystem (30) nur für Strahlung im nahen Infrarotbereich (NIR), die von der Innenseite einer Wand der Glaserzeugnisse herrührt, empfindlich ist, **dadurch gekennzeichnet, dass** der Prozessor (38) angebracht ist, um die folgenden Schritte durchzuführen:
(a) Unterteilen eines Bildes der Glaserzeugnisse (18) in zumindest zwei Messbereiche (40, 41, 42, 43, 44);
(b) Bestimmen der durchschnittlichen Intensitätswerte für die unterschiedlichen Messbereiche für aufeinander folgende Glaserzeugnisse (18);
(c) Bestimmen eines aktuellen Durchschnittswertes für zumindest zwei Messbereiche aus den für eine Anzahl von aufeinander folgend geformten Glaserzeugnissen (18) über die Zeit bestimmten, durchschnittlichen Intensitätswerten;
(d) Aufzeichnen jeder Abweichung zwischen der aktuellen Intensität oder der aktuellen durchschnittlichen Intensität und einem Bezugswert für jeden der zumindest zwei Messbereiche;
(e) Vergleichen von etwaigen Abweichungen zwischen den zumindest zwei Messbereichen;
(f) Erzeugen eines Fehlersignals im Fall von etwaigen Abweichungen.

2. Analytisches System nach Anspruch 1, **dadurch gekennzeichnet, dass** das infrarotempfindliche Messsystem (30) für Wellenlängen von zwischen 900 und 2800 Nanometern empfindlich ist.

3. Analytisches System nach einem der vorhergehenden Ansprüche, **dadurch gekennzeichnet, dass** das infrarotempfindliche Messsystem (30) zumindest einen Infrarotsensor (32) und zumindest ein Filter (34) für nahes Infrarot umfasst.

4. Analytisches System nach Anspruch 3, **dadurch gekennzeichnet, dass** die Durchlasseigenschaften des Filters (34) für nahes Infrarot von der Farbe und der spezifischen Materialzusammensetzung der Glaserzeugnisse abhängig ist.

5. Analytisches System nach Anspruch 1, **dadurch gekennzeichnet, dass** das Fehlersignal eine abweichende Glasdicke anzeigt, wenn in einem ersten Messbereich eine positive Abweichung und in einem zweiten Messbereich eine negative Abweichung auftritt.

6. Analytisches System nach Anspruch 1, **dadurch gekennzeichnet, dass** das Fehlersignal eine abweichende Glastemperatur anzeigt, wenn für alle Messbereiche eine positive Abweichung oder für alle Messbereiche eine negative Abweichung auftritt.

7. Analytisches System zum Analysieren und Überwachen eines Produktionsprozesses für Glaserzeugnisse, wobei der Produktionsprozess einen Formprozess und einen Kühlprozess umfasst, und das analytische System ein infrarotempfindliches Messsystem und einen mit diesen kommunizierenden Prozessor umfasst, wobei das infrarotempfindliche Messsystem ausgerüstet ist, um Infrarotstrahlung zu messen, die von heißen Glaserzeugnissen unmittelbar nach dem Formprozess für die Glaserzeugnisse herrührt; und der Prozessor angeordnet ist, um in den Glaserzeugnissen auf der Basis von durch das Messsystem festgelegten Informationen eine Wärmeverteilung zu bestimmen, wobei das infrarotempfindliche Messsystem (30) nur für Strahlung im nahen Infrarotbereich (NIR), die aus dem Inneren einer Wand der Glaserzeugnisse herrührt, empfindlich ist, **dadurch gekennzeichnet, dass** der Prozessor (38) angebracht ist, um die folgenden Schritte durchzuführen:
(a) Unterteilen eines Bildes der Glaserzeugnisse (18) in zumindest zwei Messbereiche (40, 41, 42, 43, 44);
(b) Bestimmen von durchschnittlichen Intensitätswerten für die unterschiedlichen Messbereiche für aufeinander folgende Glaserzeugnisse (18);
wobei der Prozessor (38) angebracht ist, um die folgenden Schritte für zumindest einen Messbereich durchzuführen:
(c) Bestimmen eines Maschinendiagramms, indem ein Kurvenbild der durchschnittlichen Intensitätswerte als Funktion der aufeinander folgenden Glaserzeugnisse (18), d.h. Stationen (14), grafisch dargestellt wird;
(d) Bestimmen einer Kühlkurve mittels Hindurchlegen einer optimalen Kurve;
(e) Aufzeichnen von etwaigen Abweichungen zwischen einem aktuellen Maschinendiagramm und der Kühlkurve;
(f) Erzeugen eines Fehlersignals im Fall von etwaigen Abweichungen.

8. Analytisches System nach Anspruch 7, **dadurch gekennzeichnet, dass** das Fehlersignal Informationen hinsichtlich eines möglichen Problems während des Formprozesses enthält.

9. Verfahren zum Analysieren und Überwachen eines Produktionsprozesses für Glaserzeugnisse, umfassend:
a) Bereitstellen einer Messeinrichtung zum Bestimmen einer Wärmeverteilung in heißen Glaserzeugnissen, wobei die Messeinrichtung (30) nur für Strahlung aus dem nahen Infrarotbereich, die aus dem Inneren einer Wand der Glaserzeugnisse herrührt, empfindlich ist.
b) Messen von Infrarotstrahlung, die von den heißen Glaserzeugnissen herrührt, bevor diese in einen Kühlofen eintreten.
c) Bestimmen einer Wärmeverteilung in Glaserzeugnissen auf der Basis der gemessenen Infrarotstrahlung;
(d) Unterteilen eines Bildes der Glaserzeugnisse (18) in zumindest zwei Messbereiche (40, 41, 42, 43, 44);
(e) Bestimmen von durchschnittlichen Intensitätswerten für die unterschiedlichen Messbereiche für aufeinander folgende Glaserzeugnisse (18);
(f) Bestimmen eines aktuellen Durchschnittswertes, zumindest für zwei Messbereiche, aus den durchschnittlichen Intensitätswerten, die für eine Anzahl von aufeinander folgend geformten Glaserzeugnissen (18) bestimmt wurden;
(g) Aufzeichnen einer etwaigen Abweichung zwischen der aktuellen durchschnittlichen Intensität und einem Bezugswert für jeden der zumindest zwei Bereiche;
(h) Vergleichen von etwaigen Abweichungen zwischen den zumindest zwei Messbereichen;
(i) Erzeugen eines Fehlersignals im Fall von etwaigen Abweichungen.

10. Verfahren nach Anspruch 9, **dadurch gekennzeichnet, dass** die Messeinrichtung (30) nur für Wellenlängen zwischen 900 und 2800 Nanometer empfindlich ist.

11. Verfahren nach Anspruch 9, **dadurch gekennzeichnet, dass** die Messeinrichtung (30) zumindest einen Infrarotsensor (32) und zumindest ein Filter (34) für nahes Infrarot umfasst.

12. Verfahren nach Anspruch 9, **dadurch gekennzeichnet, dass** das Fehlersignal eine abweichende Glasdicke anzeigt, falls in einem ersten Messbereich eine positive Abweichung und in einem zweiten Messbereich eine negative Abweichung auftritt.

13. Verfahren nach Anspruch 9, **dadurch gekennzeichnet, dass** das Fehlersignal eine abweichende Glastemperatur anzeigt, falls für alle Messbereiche eine positive Abweichung oder für alle Messbereiche eine negative Abweichung auftritt.

14. Verfahren zum Analysieren und Überwachen eines Produktionsprozesses für Glaserzeugnisse, umfassend:
a) Bereitstellen einer Messeinrichtung zum Bestimmen einer Wärmeverteilung in heißen Glaserzeugnissen, wobei die Messeinrichtung (30) nur für Strahlung aus dem nahen Infrarotbereich, die aus dem Inneren einer Wand der Glaserzeugnisse herrührt, empfindlich ist.
b) Messen von Infrarotstrahlung, die aus den heißen Glaserzeugnissen herrührt, bevor diese in einen Kühlofen eintreten;
c) Bestimmen einer Wärmeverteilung in Glaserzeugnissen auf der Basis der gemessenen Infrarotstrahlung;
(d) Unterteilen eine Bildes der Glaserzeugnisse (18) in zumindest zwei Messbereiche (40, 41 42, 43, 44);
(e) Bestimmen von durchschnittlichen Intensitätswerten für die unterschiedlichen Messbereiche für aufeinander folgende Glaserzeugnisse (18);
(f) Bestimmen eines Maschinendiagramms, indem ein Kurvenbild der durchschnittlichen Intensitätswerte als Funktion der aufeinander folgenden Glaserzeugnisse (18), d.h. Stationen (14), grafisch dargestellt wird;
(g) Bestimmen einer Kühlkurve mittels Hindurchlegen einer optimalen Kurve;
(h) Aufzeichnen von etwaigen Abweichungen zwischen einem aktuellen Maschinendiagramm und der Kühlkurve;
(i) Erzeugen eines Fehlersignals im Fall von etwaigen Abweichungen.

## Revendications

1. Système d'analyse pour analyser et surveiller un procédé de fabrication pour des produits en verre, le procédé de fabrication comprenant un processus de formage et un processus de refroidissement et le système d'analyse comprenant un système de mesure sensible à l'infrarouge et un processeur communiquant avec celui-ci, le système de mesure sensible à l'infrarouge étant équipé pour mesurer un rayonnement infrarouge provenant de produits en verre chauds immédiatement après le processus de formage pour les produits en verre et le processeur étant configuré pour déterminer une distribution de chaleur dans les produits en verre sur la base des informations déterminées par le système de mesure, ledit système de mesure sensible à l'infrarouge (30) étant sensible uniquement au rayonnement dans la région de l'infrarouge proche (NIR), provenant de l'intérieur d'une paroi des produits en verre, **caractérisé en ce que** le processeur (38) est configuré pour effectuer les étapes suivantes :
(a) subdiviser une image des produits en verre (18) en au moins deux régions de mesure (40, 41, 42, 43, 44) ;
(b) déterminer des valeurs d'intensité moyennes pour les différentes régions de mesure pour des produits en verre consécutifs (18) ;
(c) déterminer, pour au moins deux régions de mesure, une valeur moyenne actuelle à partir des valeurs d'intensité moyennes déterminées pour une pluralité de produits en verre formés consécutivement (18) au cours du temps ;
(d) enregistrer, pour chacune des au moins deux régions de mesure, un écart éventuel entre l'intensité actuelle ou l'intensité moyenne actuelle et une valeur de référence ;
(e) comparer les écarts éventuels entre les au moins deux régions de mesure ;
(f) générer un signal d'erreur en cas d'écarts éventuels.

2. Système d'analyse selon la revendication 1, **caractérisé en ce que** le système de mesure sensible à l'infrarouge (30) est sensible à des longueurs d'onde comprises entre 900 et 2800 nanomètres.

3. Système d'analyse selon l'une quelconque des revendications précédentes, **caractérisé en ce que** le système de mesure sensible à l'infrarouge (30) comprend au moins un capteur infrarouge (32) et au moins un filtre infrarouge proche (34).

4. Système d'analyse selon la revendication 3, **caractérisé en ce que** les caractéristiques de transmission du filtre infrarouge proche (34) sont dépendantes de la couleur et de la composition de matériau spécifique des produits en verre.

5. Système d'analyse selon la revendication 1, **caractérisé en ce que** le signal d'erreur est indicateur d'un écart d'épaisseur de verre si un écart positif est observé dans une première région de mesure et un écart négatif est observé dans une deuxième région de mesure.

6. Système d'analyse selon la revendication 1, **caractérisé en ce que** le signal d'erreur est indicateur d'un écart de température de verre si un écart positif est observé pour toutes les régions de mesure ou un écart négatif est observé pour toutes les régions de mesure.

7. Système d'analyse pour analyser et surveiller un procédé de fabrication pour des produits en verre, le procédé de fabrication comprenant un processus de formage et un processus de refroidissement et le système d'analyse comprenant un système de mesure sensible à l'infrarouge et un processeur communiquant avec celui-ci, le système de mesure sensible à l'infrarouge étant équipé pour mesurer un rayonnement infrarouge provenant de produits en verre chauds immédiatement après le processus de formage pour les produits en verre et le processeur étant configuré pour déterminer une distribution de chaleur dans les produits en verre sur la base des informations déterminées par le système de mesure, ledit système de mesure sensible à l'infrarouge (30) étant sensible uniquement au rayonnement dans la région de l'infrarouge proche (NIR), provenant de l'intérieur d'une paroi des produits en verre, **caractérisé en ce que** le processeur (38) est configuré pour effectuer les étapes suivantes :
(a) subdiviser une image des produits en verre (18) en au moins deux régions de mesure (40, 41, 42, 43, 44) ;
(b) déterminer des valeurs d'intensité moyennes pour les différentes régions de mesure pour des produits en verre consécutifs (18) ;
le processeur (38) étant configuré pour conduire les étapes suivantes pour au moins une région de mesure :
(c) déterminer un tracé de machine en traçant un graphique des valeurs d'intensité moyennes en fonction des produits en verre consécutifs (18), c'est-à-dire des stations (14) ;
(d) déterminer un tracé de refroidissement au moyen d'une courbe d'ajustement optimal ;
(e) enregistrer des écarts éventuels entre un tracé de machine actuel et le tracé de refroidissement ;
(f) générer un signal d'erreur en cas d'écarts éventuels.

8. Système d'analyse selon la revendication 7, **caractérisé en ce que** le signal d'erreur contient des informations sur une cause possible durant le processus de formage.

9. Procédé pour analyser et surveiller un procédé de fabrication pour des produits en verre, comprenant :
a) la disposition de moyens de mesure pour déterminer une distribution de chaleur dans des produits en verre chauds, lesdits moyens de mesure (30) étant sensibles uniquement au rayonnement dans la région de l'infrarouge proche ; provenant de l'intérieur d'une paroi des produits en verre,
b) la mesure du rayonnement infrarouge provenant des produits en verre chauds avant que ceux-ci entrent dans un four de refroidissement ;
c) la détermination d'une distribution de chaleur dans les produits en verre sur la base du rayonnement infrarouge mesuré ;
(d) la subdivision d'une image des produits en verre (18) en au moins deux régions de mesure (40, 41, 42, 43, 44) ;
(e) la détermination de valeurs d'intensité moyennes pour les différentes régions de mesure pour des produits en verre consécutifs (18) ;
(f) la détermination, pour au moins deux régions de mesure, d'une valeur moyenne actuelle à partir des valeurs d'intensité moyennes déterminées pour une pluralité de produits en verre formés consécutivement (18) ;
(g) l'enregistrement, pour chacune des au moins deux régions de mesure, d'un écart éventuel entre l'intensité moyenne actuelle et une valeur de référence ;
(h) la comparaison des écarts éventuels entre les au moins deux régions de mesure ;
(i) la génération d'un signal d'erreur en cas d'écarts éventuels.

10. Procédé selon la revendication 9, **caractérisé en ce que** les moyens de mesure (30) est sensible à des longueurs d'onde comprises entre 900 et 2800 nanomètres.

11. Procédé selon la revendication 9, **caractérisé en ce que** les moyens de mesure (30) comprennent au moins un capteur infrarouge (32) et au moins un filtre infrarouge proche (34).

12. Procédé selon la revendication 9, **caractérisé en ce que** le signal d'erreur est indicateur d'un écart d'épaisseur de verre si un écart positif est observé dans une première région de mesure et un écart négatif est observé dans une deuxième région de mesure.

13. Procédé selon la revendication 9, **caractérisé en ce que** le signal d'erreur est indicateur d'un écart de température de verre si un écart positif est observé pour toutes les régions de mesure ou un écart négatif est observé pour toutes les régions de mesure.

14. Procédé pour analyser et surveiller un procédé de fabrication pour des produits en verre, comprenant :
a) la disposition de moyens de mesure pour déterminer une distribution de chaleur dans des produits en verre chauds lesdits moyens de mesure (30) étant sensibles uniquement au rayonnement dans la région de l'infrarouge proche, provenant de l'intérieur d'une paroi des produits en verre,
b) la mesure du rayonnement infrarouge provenant des produits en verre chauds avant que ceux-ci entrent dans un four de refroidissement ;
c) la détermination d'une distribution de chaleur dans les produits en verre sur la base du rayonnement infrarouge mesuré ;
(d) la subdivision d'une image des produits en verre (18) en au moins deux régions de mesure (40, 41, 42, 43, 44) ;
(e) la détermination de valeurs d'intensité moyennes pour les différentes régions de mesure pour des produits en verre consécutifs (18) ;
(f) la détermination d'un tracé de machine en traçant un graphique des valeurs d'intensité moyennes en fonction des produits en verre consécutifs (18), c'est-à-dire des stations (14) ;
(d) la détermination d'un tracé de refroidissement au moyen d'une courbe d'ajustement optimal ;
(h) l'enregistrement des écarts éventuels entre un tracé de machine actuel et le tracé de refroidissement ;
(i) la génération d'un signal d'erreur en cas d'écarts éventuels.
